# EUROPEAN PATENT APPLICATION

(11) **EP 4 764 057 A1**
(43) Date of publication of application: **24.06.2026**
(21) Application number: 24222234.7
(22) Date of filing: 20.12.2024
(51) Int. Cl.: D06N 3/00, C12N 1/14

(54) **MYCELIUM-BASED TEXTILE MATERIAL**

(71) Applicant: BioscienZ B.V., 4817 ZB Breda (NL); B4PLastics BV, 3650 Dilsen-Stokkem (BE); Millvision B.V., 4941 VN Raamsdonksveer (NL)
(72) Inventor: van Roosmalen, Timo Josephus Petrus, 5384 MB Heesch (NL); de Laat, Wilhelmus Theodorus Antonius Maria, 4817 KW Breda (NL); Gheysens, Tom, 9890 Asper (BE); Monsegue, Luciano, 6214 PE Maastricht (NL); Thiewes, Harm Jan, 3931 CC Woudenberg (NL); Van Calker, Sander, 4817 AB Breda (NL)
(74) Representative: De Clercq & Partners

(57) **Abstract**

The invention relates to a method for producing a textile material from a mycelium. The method comprises growing a filamentous fungus in submerged culture in a medium containing a fermentable carbon-rich feedstock to generate a mycelium biomass, recovering the mycelium biomass from the medium, mixing the mycelium biomass and a fiber to form a mycelium pulp, wherein a wet strength agent is mixed with the mycelium biomass and the fiber to form the mycelium pulp or wherein a wet strength agent is added to the mycelium pulp, dewatering and drying the mycelium pulp to form a mycelium sheet and impregnating the mycelium sheet with an oil to form the textile material,. The invention further provides a textile and/or leather-like material obtainable by the method of the invention, as well as uses thereof.

## Description

### Field of the invention

The present invention relates to biobased materials, in particular mycelium-based textile and/or leather-like material. A method to produce a textile material using mycelium is provided. The invention also provides a mycelium-based textile material, and uses thereof.

### Background art

Textile materials are widely used in clothing, footwear, handbags, accessories, furniture, tools, sports equipment, wall coverings, packaging, etc. Animal leather and leather alternatives are among the world's most purchased materials.

Conventional production of natural leather relies on tanning of animal hides. Tanning is the critical process to convert the otherwise perishable skin to a stable and nondecaying material. Tanning agents include mineral salts (such as chromium sulfate) and vegetable tannins (from sources such as tree bark). Chromium-tanned leather is the most common, comprising 92% of the animal leather products in the market. However, the mineral chromium is a heavy metal, thus quite toxic to the environment as well as to the people processing it. Vegetable-tanned leather does not pollute the environment the way the chromium-based tanning process does, but the method is very water intensive. Furthermore, it takes 40-60 days to produce the leather, resulting in low productivity.

Due to these problems, there has been an increasing interest in non-animal leather alternatives. One of these alternatives is synthetic leather. Most synthetic leathers are manufactured from fossil-based feedstocks and leave a high carbon footprint, still posing a threat to the environment and human health during and after its lifespan as it takes years to degrade and releases toxic chemicals into the environment.

Non-animal leather can also be made from natural resources, including agricultural by-products such as pineapple leaves, cork, apple peels etc. or using fungal mycelium. Currently, biobased leathers made from fruits (polysaccharide-based) are typically mixed with a petroleum-based material to obtain required performance characteristics.

Mycelium-based leathers enable petroleum-free production processes, while providing high performance characteristics.

The current industrial process for producing mycelium is via solid state fermentation. The process is however time consuming (10-14 days to grow mycelium biomass), laborious and requires high energy, making the cost very high, regardless of scaling efforts. Due to the solid state method used to produce mycelium, the mycelium-based leather producers produce single sheets or would require additional process steps including disruption of the mycelium to apply a paper production process to produce the mycelium-based material. WO 2022/115541 A1 discloses a production process for composite mycelium material involving solid state cultivation of mycelium material, which is disrupted or pressed and combined with a siloxane or an aliphatic chain compound. Hence, there remains a need in the art for a sustainable alternative to natural as well as synthetic leather and other textile materials, which is obtainable in a cost-effective way.

### Summary of the invention

The present invention relates to the production of a textile material manufactured from fermenter-grown mycelium, which in a subsequent step is blended with fibers and a wet strength agent. This mixture is subsequently dehydrated and dried, preferably in a paper production process, leading to a homogeneous continuous mycelium sheet. The obtained mycelium sheet is then impregnated with an oil, preferably a natural oil or a modified natural oil, which is optionally cross-linked, leading to a flexible, leather-like, semi-finished product. In further processing step, the textile material may be coated with one or more finishing layers and/or be provided with a backing material.

Accordingly, in an aspect the present invention provides a method for producing a textile material from mycelium, said process comprising:
- growing a filamentous fungus in submerged culture in a medium containing a fermentable carbon-rich feedstock to generate a mycelium biomass;
- recovering the mycelium biomass from the medium;
- mixing the mycelium biomass and a fiber to form a mycelium pulp, wherein a wet strength agent is mixed with the mycelium biomass and the fiber to form the mycelium pulp or wherein a wet strength agent is added to the mycelium pulp;
- dewatering and drying the mycelium pulp to form a mycelium sheet; and
- impregnating the mycelium sheet with an oil to form the textile material.

The present inventors found that fungi that produce a filamentous network of mycelium that is more branched such as zygomycetes, in particular fungal strains of the Mucorales order such as *Rhizomucor, Thermomucor* and *Rhizopus* sp., dewater better and faster, rendering these fungi particularly suitable for use in the method of the invention. Also advantageously, these fungal strains can grow very fast at low pH and high temperature enabling high productivities. Moreover, due to these growth conditions, no sterile operation facilities are required.

The present inventors use a submerged fermentation process to produce mycelium biomass from diverse agri-food by-product and/or waste streams, thereby upcycling these agricultural and industrial waste streams and as such, reducing the ecological footprint. Also advantageously, since a variety of feedstocks can be used to generate the mycelium biomass, the method is very versatile and not susceptible to constraints from specific feedstock supply. In contrast to solid state methods used to produce mycelium biomass, the aqueous submerged fermentation process to harvest mycelium is highly efficient both timewise and space-wise.

In preferred embodiments, dehydration and drying of the mycelium pulp is performed using a paper production process. The submerged fermentation in combination with the dehydration and drying steps on a roll offers to produce mycelium sheets in a continuous process, in contrast to a batch wise production process based on solid state fermentation producing single sheets of mycelium leather. This renders the whole production process more efficient and more scalable, at affordable price.

By upcycling the agricultural waste and by using process steps, including the mycelium cultivating process, that require minimum energy and water, a sustainable alternative is provided for natural leather as well as petroleum-based leather.

Furthermore, as shown in the examples section, textile materials that were obtained according to the method of the present invention, showed characteristics mimicking animal-based leather, enabling their use for manufacturing leather products such as clothing, shoes, bags, accessories, coverings, automotive and/or equipment.

The invention therefore provides the following aspects and embodiments:
1. A method for producing a textile material from mycelium, said process comprising:
   - growing a filamentous fungus in submerged culture in a medium containing a fermentable carbon-rich feedstock to generate a mycelium biomass;
   - recovering the mycelium biomass from the medium;
   - mixing the mycelium biomass and a fiber to form a mycelium pulp, wherein a wet strength agent is mixed with the mycelium biomass and the fiber to form the mycelium pulp or wherein a wet strength agent is added to the mycelium pulp;
   - dewatering and drying the mycelium pulp to form a mycelium sheet; and
   - impregnating the mycelium sheet with an oil to form the textile material.
2. The method according to 1, wherein the fungus is a zygomycete, preferably a fungus from the Mucorales order.
3. The method according to 1 or 2, wherein the fungus is from a genus selected from the group consisting of: *Rhizomucor, Thermomucor* and *Rhizopus.*
4. The method according to any one of 1 to 3, wherein the fungus is *Rhizomucor pusillus.*
5. The method according to any one of 1 to 4, wherein a paper production process is used for dewatering and drying the mycelium pulp.
6. The method according to any one of 1 to 5, which is a continuous process.
7. The method according to any one of 1 to 6, wherein the fiber is provided as a fiber pulp.
8. The method according to 7, wherein the fiber pulp has a dry matter content of between 2 and 20 % (w/v), preferably between 5 and 10 % (w/v).
9. The method according to any one of 1 to 8, wherein the fiber is selected from the group comprising or consisting of: wood fiber such as hardwood fiber and softwood fiber, annual plant fiber, residual fiber from agricultural or industrial waste streams, recycled paper and/or cardboard fiber, (bio)polymeric fiber such as cellulosic fiber, cotton fiber, rayon fiber, Lyocell fiber, TENCEL^{™} fiber, polypropylene (PP) fiber, polylactic acid (PLA) fiber, thermoplastic starch (TPS) fiber, polyamide (PA) fiber, a polyester fiber such as a polyethylene terephthalate (PET) fiber, polyethylene (PE) fiber, polyhydroxyalkanoate (PHA) fiber, polyamide 6 (PA6) fiber, polyamide 6.6 (PA6.6) fiber, lignocellulosic fiber such as fiber from flax, hemp, and Miscanthus, and any combinations thereof, preferably wherein the fiber comprises lignocellulosic fiber, more preferably wherein the fiber comprises wood fiber and/or fiber from agricultural waste streams or by-products, most preferably wherein the fiber comprises softwood fiber.
10. The method according to any one of 1 to 9, wherein the mycelium biomass and the fiber are mixed in a weight ratio (mycelium biomass: fiber) of 99:1 to 50:50, preferably in a ratio of 95:5 to 50:50, more preferably in a ratio of 90:10 to 60:40, even more preferably in a ratio 85:15 to 70:30, on a dry matter base.
11. The method according to any one of 1 to 10, wherein the wet strength agent is selected from the group comprising or consisting of: polyamideamine-epichlorohydrin (PAE), polyvinylamine (PVAm), cationic glyoxalated polyacrylamide (GPAM or gPAM), chitosan, citric acid, melamine formaldehyde (MF), polyacrylamide (PAM), polyethyleneimine (PEl), lignin, cellulose nanofibrils (CNF), starch, cationic starch and any combination thereof, preferably polyamideamine-epichlorohydrin (PAE) or cationic glyoxalated polyacrylamide (GPAM).
12. The method according to any one of 1 to 11, wherein the wet strength agent is added in an amount of 0.1 to 10% by weight, preferably 0.1 to 3% by weight, more preferably 1-2% by weight relative to the mycelium pulp dry weight.
13. The method according to any one of 1 to 12, further comprising the addition of a dye to the mycelium biomass and the fiber to form the mycelium pulp, or to the mycelium pulp.
14. The method according to any one of 1 to 13, wherein the mycelium pulp has a dry matter content of between 1 and 3 % (w/v), preferably of about 2% (w/v).
15. The method according to any one of 1 to 14, wherein the mycelium sheet has a grammage of about 50 g/m² to about 1400 g/m², preferably of about 100 g/m² to about 900 g/m², more preferably of about 250 g/m² to about 750 g/m², even more preferably of about 500 g/m².
16. The method according to any one of 1 to 15, wherein the mycelium pulp is dewatered (preferably at least 800 ml starting from 1 l) at a speed of between about 5 and 1600 ml/s, preferably between about 8 and 800 ml/s, more preferably between about 15 and 250 ml/s, as measured according to ISO 5267-1.
17. The method according to any one 1 to 16, wherein the oil comprises or consists of a monounsaturated or a polyunsaturated oil, preferably a polyunsaturated oil, and/or wherein the oil is a natural oil or a modified natural oil such as a sulfonated oil or an acrylated oil, or a mixture of one or more natural oils and/or one or more modified natural oils.
18. The method according to any one of 1 to 17, wherein the oil comprises or is at least partially modified with a cross-linkable group such as a cross-linkable group selected from an acryl group, an epoxy group, a hydroxyl group, an amine group, a carboxyl group and/or a thiol group.
19. The method according to 18, further comprising a step of cross-linking the oil impregnated mycelium-sheet to form the textile material.
20. The method according to 19, wherein the cross-linking is performed with an UV treatment or a heat treatment.
21. The method according to any one 1 to 20, further comprising a step of coating the textile material with one or more finishing layers.
22. The method according to any one of 1 to 21, further comprising a step of providing the textile material with a backing material.
23. The method according to any one of 1 to 22, further comprising a step of embossing the textile material.
24. The method according to any one of 1 to 23, wherein said fungus is grown at a temperature higher than 45° C and a pH of less than 3.8.
25. The method according to any one of 1 to 24, wherein said fungus is grown under non-sterile conditions.
26. The method according to any one of 1 to 25, wherein the fermentable carbon-rich feedstock is selected from the group comprising or consisting of: a by-product or waste from agriculture or food production, silage, an organic fraction of municipal solid waste (MSW), a product of plant origin, and any combination thereof.
27. The method according to any one of 1 to 26, wherein the medium contains and/or is fed a nitrogen source, wherein preferably the nitrogen source comprises one or more of ammonia, urea and nitrate.
28. The method according to any one of 1 to 27, wherein the mycelium biomass is recovered from the medium by a mechanical dewatering technique, preferably by at least one of sieving, filtration and decantation.
29. A textile material obtainable by the method according to any one of 1 to 28.
30. The textile material according to 29, which is a leather-like material.
31. The textile and/or leather-like material according to 29 or 30 comprising mycelium biomass and fibers, wherein the mycelium biomass and the fibers are present in a weight ratio (mycelium biomass: fiber) of 99:1 to 50:50, preferably in a ratio of 95:5 to 50:50, more preferably in a ratio of 90:10 to 60:40, even more preferably in a ratio 85:15 to 70:30, on a dry matter base.
32. The textile and/or leather-like material according to any one of 28 to 30, having a grammage of about 500 g/m² to about 2800 g/m², preferably of about 500 g/m² to about 1500 g/m² or of about 550 g/m² to about 1500 g/m² or of about 750 g/m² to about 1500 g/m² or of about 1000 g/m² to about 1500 g/m², more preferably of about 550 g/m² to about 1250 g/m² or of about 750 g/m² to about 1250 g/m² or of about 1000 g/m² to about 1250 g/m².
33. The textile and/or leather-like material according to any one of 29 to 32, having a tensile strength of at least about 2.0 MPa, preferably at least about 4.0 MPa, as measured according to ISO 527.
34. The textile and/or leather-like material according to any one of 29 to 33, having an elongation of at least about 2.0%, preferably at least about 5.0%, more preferably at least about 8.0, 9.0 or 10.0%, as measured according to ISO 527.
35. The textile and/or leather-like material according to any one of 29 to 34, having a tear strength of at least 5.0 N, preferably at least 10.0 N such as of 10.0 N to 100.0 N, as measured according to ISO 13937-2.
36. Use of a textile and/or leather-like material or textile material according to any one of 29 to 35 for manufacturing clothing, shoes, bags, accessories, (wall) coverings, automotive, packaging material and/or equipment.

These and further aspects and preferred embodiments of the invention are described in the following sections and in the appended claims. The subject-matter of the appended claims is hereby specifically incorporated in this specification.

### Brief description of the figures

The teaching of the application is illustrated by the following Figures which are to be considered as illustrative only and do not in any way limit the scope of the claims.

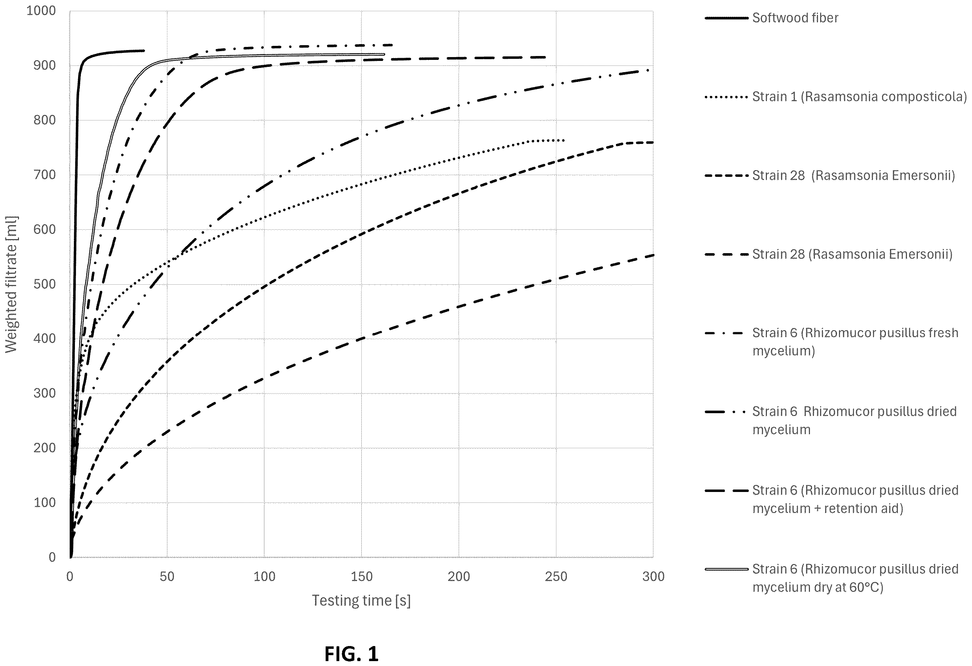

**Figure 1****: Dewatering curves of samples of softwood fiber pulp and mycelium of *Rasamsonia composticola* (strain 1), *Rasamsonia emersonii* (strain 28) and *Rhizomucor pusillus* (strain 6).** Samples of 1 l with a dry matter concentration of 2 g/l were tested in a Schopper Riegler apparatus equipped with a digital scale according to ISO 5267-1 "Determination of drainability". The graph shows filtration volume (in ml) versus time (s).

### Detailed description

The present invention will be described with respect to particular embodiments but the invention is not limited thereto but only by the claims.

When describing the invention, the terms used are to be construed in accordance with the following definitions, unless a context dictates otherwise.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

In the above passages, different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

Reference throughout this specification to "one embodiment" or "an embodiment" means that a particular feature, structure, or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while certain embodiments described herein include some but not other features included in other embodiments, combinations of features of different embodiments are meant to be within the scope of the invention, and form different embodiments, as would be understood by those in the art.

The terms "comprising", "comprises" and "comprised of" as used herein are synonymous with "including", "includes" or "containing", "contains", and are inclusive or open-ended and do not exclude additional, non-recited members, elements, or method steps. It will be appreciated that the terms "comprising", "comprises" and "comprised of" as used herein comprise the terms "consisting of", "consists" and "consists of".

As used in the specification and the appended claims, the singular forms "a", "an," and "the" include plural referents unless the context clearly dictates otherwise. By way of example, "a step" means one step or more than one step.

Unless defined otherwise, all technical and scientific terms used herein have the same meaning as is commonly understood by one of skill in the art.

When referring to the endpoints of a range, the endpoints values of the range are included. The recitation of numerical ranges by endpoints includes all integer numbers and, where appropriate, fractions subsumed within that range (e.g. 1 to 5 can include 1, 2, 3, 4 when referring to, for example, a number of elements, and can also include 1.5, 2, 2.75 and 3.80, when referring to, for example, measurements). The recitation of endpoints also includes the end point values themselves (e.g. from 1.0 to 5.0 includes both 1.0 and 5.0). Any numerical range recited herein is intended to include all sub-ranges subsumed therein.

The term "about" as used herein when referring to a measurable value such as a parameter, an amount, a temporal duration, and the like, is meant to encompass variations of +/-10% or less, preferably +/-5% or less, more preferably +/-1% or less, of and from the specified value, insofar such variations are appropriate to perform in the disclosed invention. It is to be understood that the value to which the modifier "about" refers is itself also specifically, and preferably, disclosed.

The terms "wt%," "vol%", or "mol%" refers to a weight percentage of a component, a volume percentage of a component, or molar percentage of a component, respectively, based on the total weight, the total volume of material, or total moles, which includes the component. The term "wt%" and "weight%" are used as synonyms herein.

When describing the invention, the terms used are construed in accordance with the following definitions, unless indicated otherwise.

The term 'and/or' when listing two or more items, means that any one of the listed items can by employed by itself or that any combination of two or more of the listed items can be employed. The terms 'first', 'second' and the like used in the description as well as in the claims, are used to distinguish between similar elements and not necessarily describe a sequence, either temporally, spatially, in ranking or in any other manner. It is to be understood that the terms so used are interchangeable under appropriate circumstances and that the embodiments of the invention described herein are capable of operation in other sequences than described or illustrated herein.

In an aspect, the present invention relates to a method for producing a textile material from mycelium, said process comprising:
- growing a filamentous fungus in submerged culture in a medium containing a fermentable carbon-rich feedstock to generate a mycelium biomass;
- recovering the mycelium biomass from the medium;
- mixing the mycelium biomass and a fiber to form a mycelium pulp, wherein a wet strength agent is mixed with the mycelium biomass and the fiber to form the mycelium pulp or wherein a wet strength agent is added to the mycelium pulp;
- dewatering and drying the mycelium pulp to form a mycelium sheet; and
- impregnating the mycelium sheet with an oil to form the textile material.

### Mycelium biomass

Any known submerged culture method for growing a fungus may be used for generating the mycelium biomass in the methods described herein. A submerged culture is relatively inexpensive, faster, less labor intensive, easier scalable, and on-line controllable compared to a solid state culture.

Standard methods for cultivating a mycelium typically comprise first inoculating a suitable culture medium or substrate with an inoculum of the mycelium from a selected species of fungus and incubating the mixture to grow a biomass of mycelium.

Any fungus that can be cultivated as mycelium, or any filamentous fungus may be used in the methods described herein. "Filamentous fungi" are defined herein as eukaryotic microorganisms that include all filamentous forms of the subdivision Eumycotina and Oomycota.

Suitable fungal species for use in the methods described herein include, but are not limited to: *Agaricus arvensis, Agaricus bisporus, Agrocybe brasiliensis, Amylomyces rouxii, Amylomyces sp, Armarillaria mellea, Aspergillus nidulans, Aspergillus niger, Aspergillus oryzae, Ceriporia lacerate, Coprinus comatus, Fibroporia vaillantii; Fitulina hepatica, Flammulina velutipes; Fomitopsis officinalis; Ganoderma sessile, Gonodermo tsugae, Hericium erinaceus, Hypholoma capnoides, Hypholoma sublaterium; Inonotus obliquus, Lactarius chrysorrheus; Macrolepiota procera, Morchella angusticeps; Myceliophthora thermophila, Neurospora crassa, Penicillium camembertii, Penicillium chrysogenum, Penicillium rubens, Phycomyces blakesleeanus, Pleurotus djamor; Pleurotus ostreatus, Polyporus squamosus, Psathyrella aquatica, Rhizopus microsporus, Rhizopus arrhizus, Rhizopus stolonifer, Rhizopus oryzae, Schizophyllum commune, Streptomyces venzuelae, Stropharia rugosoannulata; Thielavia terrestris and Ustilago maydis, Rhizomucor miehei, Rhizomucor pusillus, Thermomyces lanuginosus, Thermoascus thermophilus, Thielavia terricola, Thermomucor indicae-seudaticae, Rasamsonia emersonii, Rasamsonia composticola, Chaetomium thermophilum, Humicola insolens, Humicola grisea, Paecilomyces marquandii, Paecilomyces variotii, Scytalidium thermophilum, Acremonium thermophilum, Thermophymatospora fibuligera, Thielavia terrical var. Minor, Talaromyces emersonii, Talaromyces atroroseum, Trichoderma reeseii. Aspergillus fumigatus, Rhizopus chinensis, Cephalosporium eichorniae, Actinomucor elegans, Chaetomium thermoplile, Dactylomyces thermophilus, Sporotrichum thermophilae* and *Thermoascus crustaceus.*

In preferred embodiments, the fungus is a strain of a division selected from the group consisting of: Zygomycota (e.g. a strain of a fungal species selected from *Phycomyces blakesleeanus, Rhizopus microsporus, Rhizopus arrhizus, Rhizopus stolonifer, Rhizopus oryzae, Rhizomucor miehei, Rhizomucor pusillus, Thermomucor indicae-seudaticae, Rhizopus chinensis or Actinomucor elegans*), Ascomycota and Basidiomycota.

A fungus for use in the methods described herein is further preferably a strain that produces a mycelium biomass that dewaters well and fast by a mechanical dehydration technique such as mechanical pressing. Surprisingly, the inventors have found that fungal strains that produce a filamentous network of mycelium that is more branched, dewater better and faster. Without wishing to be bound by any theory, the branching of the filaments rather than the length of the filaments may determine the dewatering properties of the filamentous mycelium network. Fungal strains with suitable dewatering properties were identified among the zygomycetes. In preferred embodiments, the fungus is a strain of the Zygomycota division, more preferably a strain of the Mucoromycota division. In further preferred embodiments, the fungus is a strain of the order of the Mucorales. As shown in example 5, strains of the order of the Mucorales, such as *Rhizomucor pusillus,* were shown to dewater faster compared to *Rasamsonia* sp. strains belonging to the Asomycota division. In yet further preferred embodiments, the fungus is a strain of a genus selected from the group consisting of *Rhizomucor, Thermomucor* and *Rhizopus.* In particular embodiments, the fungus is a strain selected from a species selected from the group consisting of: *Rhizopus microsporus, Rhizopus arrhizus, Rhizopus stolonifer, Rhizopus oryzae, Rhizomucor miehei, Rhizomucor pusillus, Thermomucor indicae-seudaticae* and *Rhizopus chinensis,* preferably *Rhizomucor pusillus.*

The fermentable carbon-rich feedstock that is present in the culture medium can be any feedstock that can serve as carbon and energy source for the fungus. Such carbon-rich feedstock can be crops freshly harvested from the primary production of food sugars such as corn, sugar beet, thin juice, thick juice, sugar cane juice, or, preferably, carbon-rich side- or by-products or waste streams from agriculture and/or food production, such as e.g. sugar beet pulp, liquid C-starch from grain processing, vegetable waste from production of pealed or cut vegetables or from rejected vegetables, such as e.g. peels from potato peels and cutting residuals from French fries production, refused potato from trading, and also palm mill residues such as including palm oil mill effluent (POME) containing predominantly palm oil and palm oil fatty acids and empty fruit bunches (EFB) or palm fronds. Also feedstocks stored as a silage, so it can be processed into mycelium biomass year round, while the feedstock is harvested in a campaign such as in the case of sugar beet pulp or the leaves of potato or sugar beet. Also, silages from whole fodder beet can be used, e.g. combined with corn or whole corn or the ensilaged form of thereof, although the lignin rich corn stover is not preferred, neither sugar cane bagasse. Pentoses e.g. from lignocellulosic hydrolysates can also be used. These syrups contain mainly glucose, xylose, arabinose, mannose and galactose. Another source of raw material as fermentable carbon-rich feedstock may be the organic fraction of municipal solid waste (MSW). Also sludge from anaerobic waste water can be used. In particular embodiments, the fermentable carbon-rich feedstock is one or more of a by-product or waste from agriculture or food production, silage, an organic fraction of municipal solid waste (MSW), a product of plant origin and any combination thereof. Preferably, the fermentable carbon-rich feedstock may be one or more of sugar beet pulp, liquid C-starch from grain processing, vegetable waste from production of peeled, cut vegetables or rejected vegetables, Palm mill residues, including palm oil mill effluent (POME), empty fruit bunches (EFB) palm fronds, corn, potato, wheat, rice, cassava, sugar cane or sugar cane juice, sugar beet or sugar beet juice or thick juice, molasses, cane molasses, glucose syrups, fructose syrups and vegetable oils.

In certain embodiments, the culture medium further contains and/or is fed with a source of nitrogen. Preferably, the nitrogen source comprises one or more of (a source of) ammonia, urea and nitrate, more preferably, as a nitrogen source are the reduced form such as urea and ammonium. NH₃ or H₂NO₃ can additionally be used to control pH in the fermenter or urea can be used as a pH-independent supply of nitrogen source. Also preferred are nitrogen sources from waste streams. These include e.g. one or more of amines present in burden condensates obtained from evaporation of molasses, sugar beet or cane vinasses, vinasses from wine industry, grape residues, potato protein liquor (PPL), Corn steep liquor (CSL), ammonia from animal farm exhaust gas cleaning scrubbers, and the thin fraction of manure processing. In particular embodiments, the nitrogen source comprises one or more of ammonia, urea and nitrate. Preferably, the nitrogen source may be one or more of amines present in burden condensates obtained from evaporation of molasses, sugar beet or cane vinasses, vinasses from wine industry, grape residues, potato protein liquor (PPL), Corn steep liquor (CSL), ammonia from animal farm exhaust gas cleaning scrubbers, and the thin fraction of manure processing.

The mycelium biomass used in the methods described herein may thus be generated from waste streams, in particular low-value agri-food waste streams and/or by-products streams that are rich in carbohydrates and/or nitrogen are suitable feedstocks. Advantageously, since a variety of feedstocks can be used to generate the mycelium biomass, the method is very versatile and not dependent on a specific feedstock and therefore not susceptible to constraints from specific feedstock supply.

The incubation or growth of the fungus can occur under aerobic conditions in the presence of oxygen. The incubation temperature can be selected based on the selected fungal species. For example, the fungus may be grown at a temperature from about 20°C to about 60°C, preferably from about 20°C to about 40°C such as from about 25°C to about 40°C, from about 30°C to about 40°C, from about 35°C to about 40°C, from about 20°C to about 35°C, from about 20°C to about 30°C, or from about 20°C to about 25°C; or from about 40°C to about 60°C or more such as from about 40°C to about 55°C, from about 40°C to about 50°C, from about 40°C to about 45°C, from about 45°C to about 60°C or more, from about 50°C to about 60°C or more, or from about 55°C to about 60°C or more. Preferably, the fungus is grown at a temperature of 46, 47, 48, 49, 50, 51, 52, 53, 54 or 55° C or more. The pH of the medium can be controlled during the growth of the fungus.

Preferably, the fungus is grown at a pH of 3.8, 3.75, 3.74, 3.73, 3.72, 3.71, 3.7, 3.6, 3.5, 3.4, 3.3, 3.2, 3.1 or 3.0 or less. In particular embodiments, the fungus is grown at a temperature of at least 45°C and at a pH of 3.8 or less. Advantageously, these growth conditions of low pH and high temperature allow that no sterile conditions are required. In certain embodiments, the fungus is grown under non-sterile conditions.

In a further step of the methods described herein, the mycelium biomass is recovered from the medium. Preferably, the biomass is recovered from the medium by a mechanical separation technique, preferably by at least one of sieving, filtration and decantation. For example, but without limitation, the mycelium biomass may be recovered from the medium by at least one of rotating drum filtration, a filter press, a belt filter, a decanter centrifuge, a vibrating sieve, a drum sieve, a belt press, etc. For example, mycelium biomass may be recovered by sieving on a sieve or a screen, with 0.1, 0.5, 1 or 2 mm diameter of pores or slots. The mycelium biomass may be recovered by at least two, three or four consecutive rounds of sieving on a sieve or screen whereby a smaller diameter of pores or slots is applied in each subsequent round of sieving. For example a first round of sieving using 2 mm pore/slot diameter, followed by subsequent rounds of 1, 0.5 and/or 0.1 mm.

The dry matter content of the recovered mycelium biomass may be or be adjusted to a concentration of between 1 and 50% (w/v), such as a concentration between 1 and 20% (w/v) or between 1 and 10% (w/v), e.g. a concentration of about 5.0%, 6.0%, 7.0%, 8.0% or 9.0% (w/v). The concentration of the recovered mycelium biomass may be adjusted by suspending the recovered mycelium biomass in an aqueous solution, or by dewatering and/or drying the mycelium biomass at least partly. Dewatering of the mycelium biomass may be achieved by pressing (more of) the residual water out using e.g. compressed air using a pneuma press and/or mechanical pressing, using e.g. a belt press or a screw press. In warmer climates the mycelium biomass can simply be dried to the air (in the sun).

### Mycelium pulp

In the methods described herein, the mycelium biomass is mixed with a fiber, preferably a fiber pulp, to form a mycelium pulp.

The fiber may be combined with the mycelium biomass in an amount of from about 1 wt.% to about 50 wt.%, about 5 wt.% to about 50 wt.%, about 10 wt.% to about 50 wt.%, about 15 wt.% to about 50 wt.%, about 1 wt.% to about 45 wt.%, about 1 wt.% to about 40 wt.%, about 1 wt.% to about 35 wt.%, about 1 wt.% to about 30 wt.%, about 5 wt.% to about 45 wt.%, about 5 wt.% to about 40 wt.%, about 10 wt.% to about 40 wt.%, about 10 wt.% to about 35 wt.%, about 15 wt.% to about 35 wt.%, or about 15 wt.% to about 30 wt.%, on a dry matter base. In embodiments, the mycelium biomass and the fiber are mixed in a weight ratio (mycelium biomass: fiber) of 99:1 to 50:50, preferably in a ratio of 95:5 to 50:50, more preferably in a ratio of 90:10 to 60:40, even more preferably in a ratio 85:15 to 70:30, on a dry matter base. Without wishing to be bound by any theory, the combination with fibers may provide increased strength to the textile material, resulting in a tensile strength that can withstand practical use and/or comparable to leather and leather and leather alternatives.

The fibers can be natural fibers or synthetic fibers, or a combination thereof. Non-limiting examples of suitable fibers include wood fibers such as hardwood fibers and softwood fibers, annual plant fibers, residual fibers from agricultural or industrial waste streams, recycled paper and/or cardboard fibers, (bio)polymeric fibers such as cellulosic fibers, cotton fibers, rayon fibers, Lyocell fibers, TENCEL^{™} fibers, polypropylene (PP) fibers, polylactic acid (PLA) fibers, thermoplastic starch (TPS) fibers, polyamide (PA) fibers, polyester fibers such as polyethylene terephthalate (PET) fibers, polyethylene (PE) fibers, polyhydroxyalkanoate (PHA) fibers, polyamide 6 (PA6) fibers, polyamide 6.6 (PA6.6) fibers, lignocellulosic fibers such as fibers from flax, hemp, and Miscanthus, and any combinations thereof. Preferred fibers are lignocellulosic fiber. In particular embodiments, the fiber comprises lignocellulosic fiber, preferably the fiber comprises wood fiber and/or fiber from agricultural waste streams or by-products, more preferably the fiber comprises softwood fiber.

In preferred embodiments, the fibers are provided as a fiber pulp. The provision of fibers as a fiber pulp may advance the mixing step, e.g. resulting in a shorter mixing time, which in turn will be advantageous for the subsequent dewatering and drying step, and/or a more homogeneous mycelium pulp, which will result in a better quality textile and/or leather-like material. Preferably, the fiber pulp has a dry matter concentration of between 1 and 20 % (w/v), between 2 and 20 % (w/v), between 5 and 20 % (w/v), between 1 and 15 % (w/v), or between 1 and 10 % (w/v), preferably between 5 and 10 % (w/v). A fiber pulp can be produced according to pulping techniques well-known to the skilled person, e.g. using a pulp disintegrator.

For the formation of the mycelium pulp, the mycelium and the fiber may be pulped separately followed by a mixing step. In other embodiments, the mycelium biomass may be mixed with the fiber, preferably a fiber pulp, and said mixture may be pulped, e.g. in a pulp disintegrator. In yet other embodiments, an aqueous suspension of a mixture of the mycelium biomass and the fiber, or the fiber pulp, may be made, and said suspension may be pulped. The selection of an appropriate mixing technique belongs to the skilled person's common general knowledge. Both static and dynamic mixing techniques can be used for conducting said mixing steps.

The mycelium pulp may have a dry matter concentration between 1 and 30% (w/v). Higher dry matter concentrations would require more intensive mixing, which may result in degradation of the mycelium fibers.

The present inventors have found that for a smooth dewatering and drying of the mycelium pulp in a paper production process, the mycelium pulp that is fed to the paper production equipment preferably has a dry matter content of between 1 and 3% (w/v), more preferably of about 2% (w/v). In preferred embodiments, the mycelium pulp has a dry matter concentration of between 1 and 3% (w/v), more preferably of about 2% (w/v). In certain embodiments, the (pulped) mixture of the mycelium biomass and the fiber may be suspended in an aqueous solution to adjust the dry matter concentration of the mycelium pulp.

In embodiments, a wet strength agent may be mixed with the mycelium biomass and the fiber to form the mycelium pulp. This mixing may be conducted in one or more steps. In embodiments, the wet strength agent may be mixed with the fiber, or the fiber pulp, and this mixture may subsequently be mixed with the mycelium biomass to form the mycelium pulp. In other embodiments, the wet strength agent may be mixed with the mycelium biomass, and this mixture may subsequently be mixed with the fiber, or the fiber pulp, to form the mycelium pulp.

In other embodiments, the mycelium biomass and the fiber, or the fiber pulp, are mixed to form a mycelium pulp, and the wet strength agent is added to said mycelium pulp. Preferably, the wet-strength agent is added to the mycelium pulp before the dewatering and drying step. In other further embodiments, a wet strength agent may be added to the mycelium pulp during the dewatering and drying step, in particular during the dewatering step. For example, in embodiments wherein the mycelium pulp is dewatered and dried in a paper production process, a wet strength agent may be added to the mycelium pulp after vacuum filtration. Addition of a wet strength agent to the (partially dewatered) mycelium pulp after vacuum filtration may be achieved e.g. by spraying.

The wet-strength agent may further improve the tensile strength of the textile material and/or provide good water resistance or dry and wet strength to the textile material.

Wet-strength agents used in the paper industry may be used. Non-limiting examples of suitable wet-strength agent include polyamideamine-epichlorohydrin (PAE), polyvinylamine (PVAm), cationic glyoxalated polyacrylamide (GPAM or gPAM), chitosan, citric acid, melamine formaldehyde (MF), polyacrylamide (PAM), polyethyleneimine (PEl), lignin, cellulose nanofibrils (CNF), starch, cationic starch and any combination thereof. In embodiments, the wet-strength agent is PAE or cationic glyoxalated polyacrylamide (GPAM).

The wet-strength agent may be added in an amount of about 0.1% by weight to about 10.0% by weight, about 0.1% by weight to about 8.0% by weight, about 0.1% by weight to about 6.0% by weight, about 0.1% by weight to about 5.0% by weight, about 0.1% by weight to about 4.0% by weight, about 0.1% by weight to about 3.0% by weight, about 0.2% by weight to about 5.0% by weight, about 0.2% by weight to about 4.0% by weight, about 0.2% by weight to about 3.0% by weight, about 0.4% by weight to about 5.0% by weight, about 0.4% by weight to about 4.0% by weight, about 0.4% by weight to about 3.0% by weight, more preferably about 0.5% by weight to about 5.0% by weight, about 0.5% by weight to about 4.0% by weight, about 0.5% by weight to about 3.0% by weight, about 0.5% by weight to about 2.5% by weight, about 0.8% by weight to about 5.0% by weight, about 0.8% by weight to about 4.0% by weight, about 0.8% by weight to about 3.0% by weight, about 0.8% by weight to about 2.5% by weight, even more preferably about 1.0% by weight to about 5.0% by weight, about 1.0% by weight to about 4.0% by weight, about 1.0% by weight to about 3.0% by weight, about 1.0% by weight to about 2.5% by weight, or about 1.0% by weight to about 2.0% by weight, based on the dry weight of the mycelium pulp.

In certain embodiments, the method further comprises the addition of a dye to the mycelium biomass and the fiber to form the mycelium pulp, or the addition of a dye to the mycelium pulp. Similar as for the wet strength agent, the dye may be mixed with the fiber, or the fiber pulp, the mycelium biomass, and optionally the wet strength agent, in one or more steps to form the mycelium pulp. In other embodiments, the mycelium biomass, the fiber, or the fiber pulp, and optionally the wet strength agent, are mixed to form a mycelium pulp, and the dye is added to said mycelium pulp. Various dyes may be used to impart color to the textile and/or leather-like materials such as acid dyes, direct dyes, disperse dyes, sulfur dyes, synthetic dyes, reactive dyes, pigments (e.g. iron oxide black and cobalt blue) and natural dyes. Optionally, agents may be added to a dye solution to facilitate dye uptake and penetration into the material such as, without limitation, ammonium hydroxide and/or formic acid, or an ethoxylated fatty amine.

### Mycelium sheet

The next step in the methods described herein, is the formation of a mycelium sheet using a dewatering and drying technique(s).

The terms "dewatering" and "dehydrating" are used as synonyms herein and refer to processes and mechanical separation techniques wherein water is removed in the liquid state. The term "drying" is used herein to refer to processes where water is removed as water vapor.

Any suitable dewatering technique can be used. For example, the mycelium pulp can be dewatered by sieving; by pressing water out using e.g. compressed air using a pneuma press and/or mechanical pressing, using e.g. a belt press or a screw press; by filtration, or any combination of dewatering techniques can be used. Drying techniques are well-known to the skilled person and any drying technique can be used in the methods described herein. For example, a heating step can be applied.

In preferred embodiments, the mycelium pulp is dewatered and dried in a paper production process. In particular, the mycelium pulp may be initially dewatered by vacuum filtration, followed by pressing the mycelium sheet between rollers and finally drying the mycelium sheet on a dryer. Traditional paper production equipment (e.g. fourdrinier or rotary screen, or a foam forming paper machine) may be adapted or used to perform some, or all, of the steps disclosed herein. Advantageously, the use of a paper production process allows for a continuous production of the mycelium sheet.

The use of a fungus that dewaters well and fast as described herein, in particular a fungus of the Mucorales order such as *Rhizomucor pusillus,* enables to dewater a mycelium pulp fast in a paper production process, allowing for a highly productive method. In embodiments, the mycelium or mycelium pulp may be dewatered at a dewatering speed of between about 5 and 1.600 ml/s, preferably between about 8 and 800 ml/s, more preferably between about 15 and 250 ml/s, as measured according to ISO 5267-1 "Determination of drainability". Such dewatering speed may lead to sufficient dewatering, in particular a dewatering of at least 800 ml when starting from 1 l mycelium or mycelium pulp. In embodiments, at least 800 ml may be dewatered from 1 l of the mycelium or mycelium pulp in less than 250 s, preferably less than 200 s, more preferably less than 150 s such as less than 100 s.

The mycelium sheet may have a grammage of about 50 g/m² to about 1400 g/m², of about 50 g/m² to about 1200 g/m², of about 50 g/m² to about 1000 g/m², of about 50 g/m² to about 900 g/m², of about 100 g/m² to about 1400 g/m², of about 100 g/m² to about 1200 g/m², of about 100 g/m² to about 1000 g/m², preferably of about 100 g/m² to about 900 g/m², more preferably of about 150 g/m² to about 850 g/m², or of about 200 g/m² to about 800 g/m², or of about 250 g/m² to about 750 g/m², even more preferably of about 500 g/m².

### Textile material

Next, the mycelium sheet is treated, impregnated or soaked with an oil. The impregnation with the oil may provide softness and increased flexibility or elasticity (or reduced stiffness), resulting in increased elongation, to the textile material.

The oil may be provided in a composition, which may further comprise e.g. a solvent, an emulsifying agent, etc.

The oil can be a natural oil or a synthetic oil. With "natural oil" is meant herein an oil derived from a natural source. The term "synthetic oil" as used herein refers to an oil that is artificially synthesized. In preferred embodiments, the oil is a natural oil or a modified natural oil such as a sulfonated natural oil or an acrylated natural oil, or a mixture of one or more natural oils and/or one or more modified natural oils.

Any type of oil or fat liquor that is used in the leather industry, can be used for impregnating the mycelium sheet. Various types of natural oils may be used such as mineral, animal and plant-based oils or combinations and mixtures thereof. Non-limiting examples of suitable natural oils or fat liquors include monounsaturated oils such as olive oil, canola (rapeseed) oil, avocado oil, almond oil, macadamia nut oil, peanut oil, sesame oil, rice bran oil, apricot kernel oil, and polyunsaturated oils such as soybean oil, sunflower oil, corn oil, cottonseed oil, walnut oil, flaxseed (linseed) oil, hemp seed oil, pumpkin seed oil, grape seed oil, chia seed oil, safflower oil, castor oil, pomegranate seed oil, sea buckthorn oil, fish oil and any combinations and mixtures thereof. In certain embodiments, the oil is a polyunsaturated oil.

Preferably, the oil contains or is modified with a cross-linkable functional group such as, for example but without limitation, an acryl group, an epoxy group, a hydroxyl group, an amine group, a carboxyl group and/or a thiol group.

In particular embodiments, the oil is a mixture comprising acrylated soybean oil and a sulfonated natural oil.

Additionally, chain extenders can be added with 2 or more cross-linkable groups as mentioned above (like amines, acrylates, epoxides, hydroxyl and carboxyl groups) for extra flexibility and thus increased elongation. These chain extenders can be single chains or branching chains from natural or petrochemical origin.

Impregnation of the mycelium sheet with oil may be performed by incubating the sheet material in a solution comprising the oil/fat liquor for a predetermined period of time such as for up to 3 days, for up to 2 days or for up to 24 hours, preferably for between 1 minute and 3 days, more preferably for between 30 minutes and 24 hours, for between 30 minutes and 12 hours, for between 30 minutes and 8 hours, for between 30 minutes and 6 hours, for between 30 minutes and 3 hours, for between 30 minutes and 2 hours. The incubation time may depend on the temperature, wherein higher temperatures may favor the impregnation with the oil. In certain embodiments, the mycelium sheet may be incubated in a solution comprising the oil/fat liquor at a temperature between 4°C and 80°C, preferably between 20°C or 21°C and 80°C, more preferably between 30°C and 80°C, between 30°C and 60°C, between 30°C and 50°C, between 40°C and 80°C, between 50°C and 80°C or between 60°C and 80°C. For example, the mycelium sheet may be incubated in a solution comprising the oil/fat liquor for up to 24 hours at room temperature, for between 30 minutes and 2 hours at a temperature between 30°C and 50°C, or for between 1 minute and 1 hours such as for about 30 minutes at a temperature between 60°C and 80°C. Excess oil/fat liquor can be removed mechanically.

In preferred embodiments, the oil impregnated mycelium sheet is further treated so that a crosslink reaction occurs between the oil molecules and/or between the oil molecules and the mycelium biomass and/or between the oil molecules and the fibers. Said cross-linking may provide further strength to the textile material and/or the oil/fat liquor may be better retained in the textile material.

Standard cross-linking techniques can be used. In embodiments, cross-linking may be performed with an UV treatment or a heat treatment. Typically, a cross-linking reaction is initiated using an appropriate type of initiator as known to the skilled person. For example, suitable initiator systems for UV-based cross-linking reaction include photo initiators such as, without limitation, TPO-L, LiTPO, Irgacure, etc. Suitable initiators for heat-based cross-linking reactions may include, for example but without limitation, AIBN, benzoyl peroxide (BP), hydrogen peroxide, etc. Selection of a suitable cross-linking technique, and initiator system, may depend on the cross-linkable functional group that is present in the oil. For example, when using acrylate-based oils, cross-linking may be performed with an UV treatment using UVA light with a wavelength between 250 and 450 nm, depending on the photo initiator used. For effective cross-linking, the UV light irradiates the sheet material preferably from both sides with an intensity between 1 and 100mW/cm², preferably between 5 and 15 mW/cm², for a time between 1 and 160 min, preferably for about 30 min, depending on the photo initiator used. Sheet materials impregnated with an acrylate-based oil may also be cross-linked by a thermal treatment at between 30°C and 200°C, preferably between 80°C and 140°C, for between 1 and 120 min, preferably for about 5 min or for about 30 min, depending on the initiator used. Preferably, both sides of the sheet material are heated. Cross-linking reactions between epoxide groups, acrylate groups, hydroxyl groups, carboxyl groups and amines may be performed by a heat treatment.

In particular embodiments, the mycelium sheet is impregnated with a mixture comprising acrylated soybean oil and benzoyl peroxide, and a sulfonated natural oil, and the impregnated mycelium sheet is cross-linked by heat treatment. In particular embodiments, the mycelium sheet is impregnated with a mixture comprising acrylated soybean oil and benzoyl peroxide, a sulfonated natural oil and acetone, and the impregnated mycelium sheet is cross-linked by heat treatment.

### Post-processing steps

In further embodiments of the method described herein, the textile material may be further processed by one or more of the following:
- coating with one or more finishing layers;
- provision with a backing or supporting layer; and
- embossing.

In certain embodiments, the textile material may be coated with one or more finishing layers.

The goal of coating the textile material may be to change the color of the textile and/or leather-like material and/or change other properties. For example, by a suitable coating, the textile and/or leather-like material may be made more water repellent or get a glossy appearance. Suitable coatings may be, for example, bee wax coating, polymerizing oils, polyurethane, alginates, agar-agar or a thermoplastic material. Further suitable coatings include biofilms made from carbohydrates, proteins and/or lipids, plasticized starches, biopolymers, protein-based bioplastics made by amino acid-cross linking (e.g. starting from casein, fibroin, collagen, keratin, gluten, algae etc.), and PLA (polylactide).

The coating can be applied in one or in multiple finishing layers, at the same time or successively.

A finishing layer is typically applied by contacting aqueous solution(s) containing a polymer emulsion, crosslinkers and optionally pigment(s) and/or filler(s) on the textile material. A finishing layer may also comprise at least one monomer, and potentially at least one catalyst, the application process further comprising a step of polymerizing said monomer(s). A finishing layer may also comprise at least one polymer, at least one crosslinking agent, and potentially at least one catalyst, and the application process may further comprise a step of crosslinking said polymer(s). Typically, a 24 h time is advantageous for the applied finishing layer to stabilize and dry before proceeding to the next step. Also other known surface coating techniques such as padding, spraying or roller coating may be used. Mechanical processes such as buffing, staking and embossing, may be used as well.

In certain embodiments, an adhesive layer or primer coat may first be applied to the textile material to promote adhesion of the finishing layer(s). The adhesive layer may comprise an aqueous dispersion of alcohol and polymer, or a polymer (e.g. PU). After drying of the adhesive layer, a subsequent coat or finishing layer(s) may be applied. At this stage, coloring agents, pigments or fillers may be added in the finishing layer solution in order to bring or adjust color of the textile and/or leather-like material.

In embodiments, the textile and/or leather-like material may be provided with a backing layer. The backing layer may provide a mechanical support to the textile and/or leather-like material, thereby improving mechanical properties, such as tear strength, of the textile and/or leather-like material or textile material. The backing layer may include natural fabric or a synthetic backing material such as, for example, polyethylene terephthalate (PET), nylon, acrylic, other thermoplastics, or some combination thereof.

The backing layer may be applied to the textile material by first applying an adhesive agent, e.g. glue, onto a first surface of the textile material and/or a first surface of the backing layer and applying pressure on at least one of the backing layer and the textile material to press the backing layer and the textile material against each other, e.g., under heat.

### Textile and/or leather-like material

Also provided herein is a textile material obtainable by the method of the invention.

The term "textile" as used herein generally includes various fiber-based materials, including fibers, yarns, filaments, threads, different fabric types, etc.

In preferred embodiments, the textile material is a leather-like material. As used herein, the term "leather-like" material refers to a textile material that has a texture, appearance and/or one or more (mechanical) properties resembling leather so that it can be used to replace leather.

The textile and/or leather-like material may have a grammage of about 500 g/m² to about 2800 g/m², or of about 500 g/m² to about 2600 g/m², or of about 500 g/m² to about 2400 g/m², or of about 500 g/m² to about 2200 g/m², or of about 500 g/m² to about 2000 g/m², or of about 500 g/m² to about 1800 g/m², or of about 500 g/m² to about 1600 g/m², preferably of about 500 g/m² to about 1500 g/m², or of about 550 g/m² to about 1450 g/m², or of about 600 g/m² to about 1400 g/m², or of about 700 g/m² to about 1300 g/m², preferably of about 750 g/m² to about 1250 g/m², or of about 800 g/m² to about 1200 g/m². In embodiments, the textile and/or leather-like material may have a grammage of more than 500 g/m², preferably more than 550 g/m² such as more than 600, 650, 700, 750, 800, 850, 900 or 950 g/m², or even more than 1000 g/m² such as from about 1000 g/m² to about 1500 g/m².

The textile and/or leather-like material comprises mycelium biomass and fibers, preferably wherein the mycelium biomass and the fibers are present in a weight ratio (mycelium biomass: fiber) of 99:1 to 50:50, preferably in a ratio of 95:5 to 50:50, more preferably in a ratio of 90:10 to 60:40, even more preferably in a ratio 85:15 to 70:30, on a dry matter base.

The textile and/or leather-like material obtained with the method of the present invention has mechanical properties, which can withstand practical use such as being used in a leather product such as, without limitation, clothing, shoes, bags, accessories, coverings, automotive and/or equipment. The textile and/or leather-like material is a sustainable alternative to natural as well as synthetic, petroleum-based leathers.

In embodiments, the textile and/or leather-like material has a tensile strength of at least about 1.0 MPa, preferably at least about 2.0 MPa, at least about 3.0 MPa, or at least about 4.0 MPa, more preferably at least about 5.0 MPa, at least about 6.0 MPa, at least about 7.0 MPa, at least about 8.0 MPa or at least about 9.0 MPa, at least about 10.0 MPa, at least about 11.0 MPa, at least about 12.0 MPa, at least about 13.0 MPa, at least about 14.0 MPa or at least about 15.0 MPa.

In embodiments, the textile and/or leather-like material has an elongation of at least about 2.0%, preferably at least about 4.0%, about 5.0%, about 6.0% or about 7.0%, more preferably at least about 8.0, 9.0 or 10.0%.

The tensile strength and elongation of the textile and/or leather-like material were measured according to "ISO 527".

In embodiments, the textile and/or leather-like material has a tear strength of at least about 1.0 N. Tear strength may be improved by providing the textile material with a backing. In particular embodiments, the textile and/or leather-like material, in particular the textile and/or leather-like material or textile material provided with a backing, has a tear strength of at least 5.0 N or at least 8.0 N, preferably at least 10.0 N such as of 10.0 N to 100.0 N.

The tear strength of the textile and/or leather-like material was measured according to "ISO 13937-2".

### Uses

As noted above, the mechanical properties of the textile material obtained by the method of the present invention are suitable to apply it for manufacturing textile and leather products. Accordingly, a further aspect relates to use of a textile material as described herein for producing textile and/or leather products such as, without limitation, clothing, shoes, bags, accessories, (wall) coverings, packaging material, automotive and/or equipment.

The following examples serve to merely illustrate the invention and should not be construed as limiting its scope in any way. While the invention has been shown in only some of its forms, it should be apparent to those skilled in the art that it is not so limited, but is susceptible to various changes and modifications without departing from the scope of the invention.

### Examples

### Example 1: Making a handsheet in the Rapid-Köthen handsheet former from a mixture of Rhizomucor pusillus and softwood fiber.

50 g of dry matter *Rhizomucor pussilis* powder was suspended in 1 L of tap water, and the whole was blended for 2 minutes resulting in a 5% (w/v) mycelium suspension/pulp.

A 10% (w/w) softwood fiber pulp was made, using a pulp disintegrator (Lorentzen & Wettre AB) according to Tappi norm T-205.

A mixture of 85 wt% mycelium and 15 wt% softwood fiber pulp was then prepared. Briefly, the mycelium and softwood fiber pulp were combined and supplemented to 2 L with tap water. The mixture was disintegrated at 30,000 rpm in a pulp disintegrator.

The obtained pulp suspension was transferred to a 2 L measuring cup and 15 kg/ton polyamideamine-epichlorohydrin (PAE) was added, followed by stirring for at least 1 min.

Mycelium sheets were made using a Rapid-Köthen sheet former (Labor Geräte Service, LGS-RK2A TH) according to the manufacturer's instructions. A polyester screen cloth was applied to the sieve of the sheet former to facilitate the release of the mycelium sheet. The mycelium sheets were dried for 2x5 min, and subsequently post-dried for at least 1 hour at 105 °C on a Speed drier (Emerson Apparatus, Model 145). After the sheets were conditioned for 1 day at room temperature and 50% relative humidity, part of sheets were impregnated with a fat liquor to obtain a textile material.

Both the mycelium sheet and the textile material were analysed on mechanical properties, and the results are shown in Table 1.

**Table 1: Mechanical properties of a mycelium sheet and a textile material obtainable by a method according to an embodiment of the invention using dried Rhizomucor pusillus and softwood fiber. Mechanical properties were measured according to the standard ISO 527.**

| | Mycelium sheet | Textile material |
|---|---|---|
| Grammage (g/m²) | 259 | 583 |
| Tensile strength (MPa) | 15.5 | 3.5 |
| Elongation (%) | 1.8 | 12.2 |

### Example 2: Making a handsheet in the Rapid-Köthen handsheet former from a mixture of Rhizomucor pusillus mycelium and softwood fiber.

*Rhizomucor pusillus* was grown at µ of 0.4 hr⁻¹ on a defined medium containing glucose and ammonium as carbon and nitrogen source, respectively in a fed batch process at 49.5 °C and pH 3.5, under aerobic conditions at excess oxygen (more than 0.5 mg/L) by aerating and stirring. The mycelium biomass was sieved and pressed over 250 µm sieve and vacuum packed and stored at 4 °C. The dry matter content of the mycelium obtained was 14.0% (w/v).

A 10% (w/v) softwood fiber pulp was made, using a pulp disintegrator according to the manufacturer's instructions.

85 wt.% mycelium and 15 wt.% softwood fiber pulp were combined and supplemented to 2 l with tap water and the mixture was disintegrated at 30,000 rpm in a pulp disintegrator.

The obtained mycelium pulp suspension was then transferred to a 2 I measuring cup, 15 kg/ton (1.5 %) PAE was added, followed by stirring for at least 1 min.

A mycelium sheet was made using the Rapid-Köthen sheet former according to the manufacturer's instructions. A polyester screen cloth was applied to the sieve of the sheet former to facilitate the release of the sheet. The sheet was dried for 2 x 5 min, and then further dried for at least 1 hour at 105 °C on a drying table. After the sheets were conditioned for 1 day, the mechanical properties were determined according to the standard ISO 527 (Table 2).

Next, the mycelium sheet was impregnated with a fat liquor to obtain a textile material. The mechanical properties of the textile material are shown in Table 2.

**Table 2: Mechanical properties of a mycelium sheet and a textile material obtainable by a method according to an embodiment of the invention using Rhizomucor pusillus mycelium and softwood fiber. Mechanical properties were measured according to the standard ISO 527.**

| | Mycelium sheet | Textile material |
|---|---|---|
| Grammage (g/m²) | 443 | 1012 |
| Tensile strength (MPa) | 19.8 ± 0.2 | 4.7 |
| Elongation (%) | 1.7 | 10.5 |

Hand sheets were made as described above, but using a dried mycelium of *Rhizomucor pusillus,* which was brought back into suspension. The mechanical properties of the dried mycelium-based mycelium sheet before and after impregnation are shown in Table 3.

**Table 3: Mechanical properties of a sheet material using dried Rhizomucor pusillus mycelium before and after impregnation with fat liquor. Mechanical properties were measured according to the standard ISO 527.**

| | Mycelium sheet | Textile material |
|---|---|---|
| Grammage (g/m²) | 492 | 1046 |
| Tensile strength (MPa) | 17.4 ± 1.7 | 4.5 ± 0.5 |
| Elongation (%) | 1.7 | 10.4 |

### Example 3: Making a textile material on a pilot paper machine from a mixture of mycelium and a paper fiber blend of softwood and hardwood

500 I mycelium pulp with a dry matter content of about 2% (w/v) was prepared of mycelium and softwood/ hardwood fiber in a stirring vessel. In particular, the following raw materials were used:
7.35 kg of air-dry mycelium powder (dried *Rhizomucor pusillus* mycelium),
3.30 kg air-dry softwood and hardwood fiber
750 g PAE

Briefly, in a 35 I pulper with pulper rotor and 1,5 kW motor, 4 x 825 g paper was weighed air dry and pulped in warm water for 10 min to obtain a softwood/hardwood fiber pulp. The mycelium powder was soaked in warm water and pulped in batches of 15 I (1000 g air dry mycelium powder in 5 I warm water) for about 10 min until no more lumps were observed. The pulped mycelium was mixed with the fiber pulp in a dry weight ratio of 70:30. The obtained mycelium pulp was then diluted to a 2 % (w/v) mycelial pulp suspension, and 750 g PAE was added.

Subsequently, two runs were performed on a Pilot Paper Machine with PAMA headbox and wire section. Rebuild double press section with 3 felts and a rebuild 3 cylinder large drying section with respectively 1x water a 2 x hot air contact dying. The speed of the machine was 2,2 m/min and the working width was 270mm:
Run 1: flow mycelium pulp suspension: ± 30-33 l/min. The mycelium sheets, on length of speed dryer after press, were put away under plastic and dried in the following days. Approximately 250 I of the mycelium pulp was consumed.
Run 2: flow was increased to 38-40 l/min. Since the mycelium pulp did not come off the screen dry, but remained too wet in the press, the last 150 I were processed at 30-33 l/min.

While processing the mycelial pulp on the paper machine, the following observations were made: The flow was constant. The vacuum batch indicated -500/-600m bar and formation went well. Dry matter content after pressing was measured in run 1 at 56%.

The mycelium sheets were dried and conditioned for at least 24 h, and the mechanical properties were determined according to the standard ISO 527 (Table 4).

Next, the mycelium sheets were impregnated with a fat liquor and conditioned to obtain a textile material. The mechanical properties of the textile material are shown in Table 4.

**Table 4: Mechanical properties of a mycelium sheet and textile material obtainable by a method according to an embodiment of the invention using mycelium and hardwood/softwood fiber. Mechanical properties were measured according to the standard ISO 527.**

| | Mycelium sheet | Textile material |
|---|---|---|
| Grammage (g/m²) | 813 | 1080 |
| Tensile strength (MPa) | 12.0 | 5.3 |
| Elongation (%) | 1.3 | 8.3 |

### Example 4: Effect of applying a backing layer

The textile materials from a pilot paper run of example 3 were used for testing the effect of a backing layer.

Different backing materials were used: both natural materials and synthetic polymer materials. The different backing materials were bonded to the textile materials using a d4 construction urethane adhesive (Bison Porefessional). After the backing was adhered, the textile material sheets were conditioned for 1 week.

Samples measuring 16 cm (length) by 5 cm (width) were cut from these sheets with backing. Subsequently, the samples were cut in the middle on the wide side for a length of 10 cm. Tear-through resistance measurements were performed according to the standard ISO 13937-2. The results are shown in Table 5.

**Table 5. Improvement of tear-through resistance by applying a backing layer to the textile material.**

| Backing materials: | Tear-off force (N) |
|---|---|
| No backing, but post-treatment | 1,2 |
| Unbleached cotton | 12,1 |
| Jersey fabric | 7,2 |
| CS 100 polyester | 38,9 |
| Cotton sheet | 18,8 |
| C100 PA / PET | 55,9 |

### Example 5: Dewatering characteristics of fungal strains

Dewatering tests have been performed with a Schopper Riegler apparatus enhanced with a digital scale. Normally the apparatus is used for determination of pulps beating degree in °SR, but equipped with a scale, dewatering curves can be produced as shown in Figure 1.

Samples of softwood fibre pulp or mycelium of *Rasamsonia composticola* (strain 1), *Rasamsonia emersonii* (strain 28) or *Rhizomucor pusillus* (strain 6) were tested. For *Rhizomucor pusillus* (strain 6), freshly grown mycelium and mycelium that was dried and resuspended were tested. Samples of 1 l with a dry matter concentration of 2 g/l were tested. Also, the effect of aids (e.g. PAC (flocculant), PEI and PAM (retention aids)), and processing temperature (dewatering at 60°C) have been tested.

Dewatering characteristics varied between the strains, with the *Rhizomucor pusillus* strain showing better, faster dewatering compared to the *Rasamsonia* strains (Fig. 1). Temperature and process aids slightly improved dewatering characteristics of the mycelium (Fig. 1).

**Table 6. Dehydration speed of 1l samples of softwood fibre pulp, and mycelium of Rasamsonia composticola (strain 1), Rasamsonia emersonii (strain 28) and Rhizomucor pusillus (strain 6), either freshly grown or dried and re-suspended mycelium, at 2 g/l, as determined from the dewatering curve (Fig. 1) obtained from a Schopper Riegler apparatus enhanced with a digital scale according to ISO 5267-1 "Determination of drainability". Dehydration speed of softwood fibre pulp was measured as a reference. t.v.n.r.: Target value of 800 ml filtrate not reached; n.d. not determined.**

| Sample | Time to 500 ml | Dehydration speed | Time to 800ml | Dehydration speed |
|---|---|---|---|---|
| | (s) | (ml/s) | (s) | (ml/s) |
| Softwood fibre pulp | 2,5 | 720 | 3,8 | 211 |
| *Rasamsonia composticola* BZF90 | 33 | 55 | t.v.n.r. | n.d. |
| *Rasamsonia emersonii* BZF102 | 102 | 18 | t.v.n.r. | n.d. |
| *Rasamsonia emersonii* BZF103 | 240 | 8 | t.v.n.r. | n.d. |
| *Rhizomucor pusillus* (fresh mycelium) | 11 | 164 | 34,5 | 23 |
| *Rhizomucor pusillus* (dried mycelium) | 43 | 42 | 174 | 5 |

### Example 6: Cross-linking of oil in mycelium sheets

Mycelium sheets were impregnated with a mixture of natural oil(s)/fat liquor(s) containing cross-linkable groups and cross-linked by heat treatment. Tensile strength and elongation were evaluated according to the standard ISO 527.

### Materials

- mycelium sheet strips as prepared in example 3 (dimensions: 15mm x 70mm)
- acrylated soybean oil (ASO)
- sulfonated natural oil / fat liquor
- benzoyl peroxide (BP)

### Methods

Besides two blanks/references: the dry and the fat liquor references, 3 cross-linking mixtures were tested.

The dry reference sheets were tested in a dry state without any treatments (no impregnation in natural oil/fat liquor; no cross-linking) and the fat liquor reference was made by sheets impregnated into sulphonated natural oil/fat liquor (no cross-linking).

The three cross-linking treatments consisted of:
- Procedure 1:
   Sheets impregnated with sulphonated natural oil/fat liquor, acrylated soybean oil and benzoyl peroxide, and cross-linked by heat treatment
- Procedure 2:
   Sheets impregnated with acrylated soybean oil and benzoyl peroxide, and acetone and cross-linked by heat treatment
- Procedure 3
   Sheets impregnated with sulphonated natural oil/fat liquor, acrylated soybean oil and benzoyl peroxide, and acetone and cross-linked by heat treatment

**Table 7. Mixtures of natural oils/fat liquors**

| Ingredient | Procedure 1 | Procedure 2 | Procedure 3 |
|---|---|---|---|
| acrylated soybean oil (ASO) | 56 wt.% | 78 wt.% | 46 wt.% |
| Benzoyl peroxide | 1 wt.% | 1 wt.% | 1 wt.% |
| Sulfonated natural oil/fat liquor | 43 wt.% | - | 10 wt.% |
| Acetone | - | 21 wt.% | 43 wt.% |

Ten (10) strips of mycelium sheet were prepared for each procedure indicated above (see Table 7). In a 50 ml beaker, the components of the mixtures shown in Table 7 were thoroughly mixed by hand using a spatula until homogenous solutions were obtained. Each of the resulting mixtures were poured into petri dishes, ensuring that the mycelium strips were fully submerged and uniformly impregnated with the solutions. The mycelium strips were allowed to remain in the solutions for a period of 3 hours, while turning them over every hour to ensure even exposure to the solution. After 3 hours, the strips were removed from the solutions and pat dried using a paper towel. The treated mycelium sheets were then placed in an oven at 80°C for 30 minutes. Thereafter, they were taken out of the oven and allowed to acclimatize at room temperature for 2 days before testing in a tensile tester.

### Results

The results are shown in Table 8. When the mycelium sheet was tested in a dry state without any treatments the tensile strength was quite high (10 MPa) but the elongation was very low (1.3%). When the sheet was impregnated with sulphonated natural oil/fat liquor (fat liquor reference/ no cross-linking), it became very flexible and the elongation went to 6.4% but the tensile strength decreased significantly to half (4.5MPa).

When the sheets were treated according to procedure 2, the tensile strength increased significantly (17MPa), but the elongation stayed almost the same as the dry reference sheet. After impregnating the mycelium sheet with a mixture of sulphonated natural oil/fat liquor, acrylated soybean oil and benzoyl peroxide and cross-linking the impregnated sheet according to procedure 1, the elongation was more similar to the sheet impregnated with sulphonated natural oil/fat liquor (fat liquor reference) and the strength was significantly increased (7.5MPa). Upon combining procedure 1 and procedure 2 into procedure 3, we got even better results, with tensile strength values equal to the dry reference sheet and an elongation 1/3 lower than the sulphonated natural oil/fat liquor impregnated sheet (fat liquor reference).

**Table 8. Mechanical properties indicating the tensile strength and the elongation of the dry reference, the fatliquor reference, and the mycelium sheet samples treated according to procedures 1, 2 and 3.**

| | Dry reference | Fat liquor reference | Procedure 1 | Procedure 2 | Procedure 3 |
|---|---|---|---|---|---|
| Tensile strength (MPa) ± standard deviation | 10.2 ± 3.0 | 4.5 ± 0.3 | 7.5 ± 0.7 | 17.6 ± 0.9 | 9.9 ± 1.0 |
| Elongation (%) ± standard deviation | 1.3 ± 0.3 | 6.4 ± 0.5 | 5.9 ± 0.8 | 1.7 ± 0.2 | 4.5 ± 0.6 |

### Example 7: Making a handsheet in the Rapid-Köthen handsheet former from a mixture of Rhizomucor pusillus mycelium and softwood fiber using gPAM as wet strength agent

900 ml mycelium (using dried *Rhizomucor pusillus*) with a dry matter content of about 5% (w/v) was prepared.

In the parallel, a 10% (w/v) softwood fiber pulp was made, using a pulp disintegrator (Lorentzen & Wettre AB) according to Tappi norm T-205.

A mixture of 85 wt.% mycelium and 15wt.% softwood fiber pulp was then prepared. Briefly, the mycelium and the softwood fiber pulp were combined and supplemented to 2 I with tap water. The mixture was disintegrated at 30,000 rpm in a pulp disintegrator.

The obtained pulp suspension was transferred to a 2 I measuring cup and 15 kg/ton polyamideamine-epichlorohydrin (PAE) or 15 kg/ton cationic (medium or high cationic) glyoxalated polyacrylamide (gPAM)) was added ending up in a handsheet of about 500 g/m², followed by stirring for at least 1 minute.

A mycelium sheet was made using a Rapid-Köthen sheet former (Labor Geräte Service, LGS-RK2A TH) according to the manufacturer's instructions. A polyester screen cloth was applied to the sieve of the sheet former to facilitate the release of the mycelium sheet. The mycelium sheet was dried for 2x 5 min, and subsequently post-dried for at least 1 hour at 105°C on a Speed drier (Emerson Apparatus, Model 145). After the sheets were conditioned for at least 1 day at room temperature, the mechanical properties of the mycelium sheets were determined according to standard ISO 527 (see Table 9).

In the parallel, mycelium sheets were impregnated with a sulfonated natural oil / fat liquor to obtain a textile material. The mechanical properties of the impregnated mycelium sheets are also shown in Table 9.

**Table 9: Mechanical properties of the mycelium sheets and textile materials obtainable by a method according to an embodiment of the invention using dried Rhizomucor pusillus mycelium and softwood fiber. Mechanical properties were measured according to the standard ISO 527.**

| Wet strength agents: | Mycelium Sheet Stiffness (MPa) | Mycelium Sheet Strength (MPa) | Mycelium Sheet Elongation (%) | Textile Material Stiffness (MPa) | Textile Material Strength (MPa) | Textile Material Elongation (%) |
|---|---|---|---|---|---|---|
| PAE | 1490 | 15,5 | 1,8 | 162 | 6,0 | 9,5 |
| gPAM high cat | 1187 | 13,4 | 2,2 | 65 | 2,3 | 9,4 |
| gPAM med cat | 1359 | 16,9 | 2,3 | 146 | 4,5 | 9,3 |

## Claims

1. A method for producing a textile material from mycelium, said process comprising:
- growing a filamentous fungus in submerged culture in a medium containing a fermentable carbon-rich feedstock to generate a mycelium biomass, wherein the fungus is a zygomycete;
- recovering the mycelium biomass from the medium;
- mixing the mycelium biomass and a fiber to form a mycelium pulp, wherein a wet strength agent is mixed with the mycelium biomass and the fiber to form the mycelium pulp or wherein a wet strength agent is added to the mycelium pulp;
- dewatering and drying the mycelium pulp to form a mycelium sheet using a paper production process; and
- impregnating the mycelium sheet with an oil to form the textile material.

2. The method according to claim 1, wherein the fungus is a fungus from the Mucorales order, preferably a fungus selected from the group consisting of: *Rhizomucor, Thermomucor* and *Rhizopus,* more preferably *Rhizomucor pusillus.*

3. The method according to claim 1 or 2, wherein the fiber is provided as a fiber pulp, preferably wherein the fiber pulp has a dry matter content of between 2 and 20 % (w/v), preferably between 5 and 10 % (w/v).

4. The method according to any one of claims 1 to 3, wherein the mycelium biomass and the fiber are mixed in a weight ratio (mycelium biomass: fiber) of 99:1 to 50:50, preferably in a ratio of 95:5 to 50:50, more preferably in a ratio of 90:10 to 60:40, even more preferably in a ratio 85:15 to 70:30, on a dry matter base.

5. The method according to any one of claims 1 to 4, wherein a dye is mixed with the mycelium biomass and the fiber to form the mycelium pulp or wherein a dye is added to the mycelium pulp.

6. The method according to any one of claims 1 to 5, wherein the wet strength agent is added in an amount of 0.1 to 10% by weight, preferably 0.1 to 3% by weight, more preferably 1-2% by weight relative to the mycelium pulp dry weight

7. The method according to any one of claims 1 to 6, wherein the mycelium pulp has a dry matter content of between 1 and 3 % (w/v), preferably of about 2% (w/v).

8. The method according one of claim 1 to 7, wherein the dewatering speed is between about 5 and 1.600 ml/s, preferably between about 8 and 800 ml/s, more preferably between about 15 and 250 ml/s, as measured according to ISO 5267-1.

9. The method according to any one of claims 1 to 8, wherein the oil comprises a monounsaturated or a polyunsaturated oil, preferably a polyunsaturated oil, and/or wherein the oil is a natural oil or a modified natural oil such as a sulfonated oil, or any mixture thereof.

10. The method according to any one of 1 to 9, further comprising a step of cross-linking the oil impregnated mycelium-sheet to form the textile material, wherein the oil comprises or is at least partially modified with a cross-linkable group such as a cross-linkable group selected from an acryl group, an epoxy group, a hydroxyl group, an amine group, a carboxyl group and/or a thiol group.

11. The method according to any one of claims 1 to 10, further comprising one or more of the following:
- coating the textile material with one or more finishing layers;
- embossing the textile material; and
- providing the textile material with a backing material.

12. The method according to any one of claims 1 to 11, wherein the fermentable carbon-rich feedstock is selected from the group comprising or consisting of: a by-product or waste from agriculture or food production, silage, an organic fraction of municipal solid waste (MSW), a product of plant origin, and any combination thereof.

13. The method according to any one of claims 1 to 12, wherein said fungus is grown under non-sterile conditions at a temperature higher than 45° C and a pH of less than 3.8.

14. A textile material obtainable by the method according to any one of claims 1 to 13, preferably wherein said textile material is a leather-like material.

15. The textile and/or leather-like material according to claim 14, which is **characterized by** one or more of:
- having a grammage of about 500 g/m² to about 2800 g/m², preferably of about 500 g/m² to about 1500 g/m², more preferably of about 550 g/m² to about 1500 g/m²;
- having a tensile strength of at least about 2.0 MPa, preferably at least about 4.0 MPa, as measured according to ISO 527;
- having an elongation of at least about 2.0%, preferably at least about 5.0%, as measured according to ISO 527; and
- having a tear strength of at least 5.0 N, preferably at least 10.0 N such as of 10.0 N to 100 N, as measured according to ISO 13937-2.

16. Use of a textile and/or leather-like material according to claim 14 or 15 for manufacturing clothing, shoes, bags, accessories, coverings, packaging material, automotive and/or equipment.
